# EUROPEAN PATENT APPLICATION

(11) **EP 1 790 335 A1**
(43) Date of publication of application: **30.05.2007**
(21) Application number: 05024815.2
(22) Date of filing: 14.11.2005
(51) Int. Cl.: A61K 9/51, A61K 9/16, A61K 47/16, A61K 47/34, A61K 33/00, A61P 35/00

(54) **Composition and its use for the manufacture of a medicament for treating, prophylactically treating, preventing cancer and/or infections in the urinary tract**

(71) Applicant: NOLabs AB, 252 21 Helsingborg (SE)
(72) Inventor: Peters, Tor, 8703 Erlenbach (CH); Wiklund, Peter, 181 60 Lidingö (SE)
(74) Representative: Krahbichler, Erik

(57) **Abstract**

A composition is provided that allows for treatment of cancer, prophylactic treatment, and treatment of infection in the urinary tract- The composition elutes nitric oxide (NO) in a therapeutic dose.

## Description

### Field of the Invention

This invention pertains in general to the field of treatment of cancer in the urinary tract, prophylactic treatment of the urinary tract, and therapy of infection in the urinary tract. More particularly the invention relates to a composition for treatment and prevention of cancer in the urinary tract, and a process for manufacturing of said composition, involving the use of nitric oxide (NO). Also, the present invention pertains to the use of NO for treatment and/or prevention of cancer in the urinary tract.

### Background of the Invention

Cancer in the urinary bladder is the fourth most common malignancy among men, and the eighth most frequent among women. An average of approximately 300,000 new cases of cancers in the urinary bladder are diagnosed world wide every year. Of these are 90% of "Transitional Cell Carcinoma" (TCC) type, originating in the epithelial cells (the internal lining) of the bladder wall. When the tumour is limited to this layer, it is called "superficial" cancer in the urinary bladder. This type of cancer tends to recur despite surgery and treatments according to the prior art.

Today, the most common way of treating cancer in the urinary bladder is to surgically remove the tumour(s) under anesthesia (partial or general). Most of the time such surgery is performed through the urethra of the patient. If numerous tumours are present the physician often is compelled to perform many such surgeries or a more extensive operation, in which a partial or complete removal of the urinary bladder is required. Especially if the cancer has migrated to the muscle layer of the urinary bladder. Surgery, no matter in what extent, is always a very trying and cumbersome experience for the body and patient on which the surgery is performed. It always results in some time of recovery and period of convalescence. After having ascertained the type of tumour present in the urinary bladder through evaluation of the performed surgery, the urinary bladder may be flushed with chemotherapeutic materials to thereby destroy cancer cells that were not removed during surgery. This is done to kill off remaining cancer cells and to prevent growth of such remaining cancer cells. The use of chemotherapeutic materials involve the problem that the chemotherapeutic materials not only destroys cancer cells but also healthy cells, which leads to deteriorated immune defence of the patient being treated.

It is known that nitric oxide (NO) provides an alternative to conventional therapies, such as antibiotics. Nitric oxide is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc.

NO is also known to have an anti-pathogenic effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, resulting in fewer and milder adverse effects as for instance many anti-cancer drugs.

However, due to the short half-life of NO, it has hitherto been very hard to treat cancers for a sufficient period of time to obtain results. This is because NO is actually toxic in high concentrations and has negative effects when applied in too large amounts to the body. NO is also a vasodilator, and too large amounts of NO introduced into the body will cause a marked reduction of the blood pressure that may result in a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

Urine is a unique environment in respect of treatment with nitric oxide, since the endoxidation product of nitric oxide is NO₂⁻. In almost all other biological tissues nitric oxide is oxidized to NO₃⁻, since there is haemoproteins, such as haemoglobin, present. This effect results in that very high concentrations of nitric oxide may be obtained during long periods of time in the urinary bladder, since the nitric oxide that is eluted in urinary bladder is first oxidized to NO₂⁻, which in return is reduced back to nitric oxide. This effect may be observed already at a pH of 7.5, but is strongly amplified at lower pH.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible before and after the release of nitrogen oxide.

Other example for NO eluting polymers are given in US-5,770,695, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be spun onto the surface of medical devices to be permanently implanted in the body, such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is silent concerning an improvement of present technology in respect of treatment of cancer in the urinary bladder with nitric oxide.

Hence, an improved composition for the treatment and/or prevention of cancer in the urinary tract, prophylactic treatment of the urinary tract, and therapy of infection of the urinary tract, which composition presents the possibility to treat and/or prevent such disorders, does not involve a surgical step, does not involve the use of chemotherapeutic materials, does not cause resistance against the active pharmaceutical substance, is easy to apply, provides improved circulation in form of a vasodilating effect, provides a painless treatment, has fast inset of treatment effect, would be advantageous.

### Summary of the Invention

Accordingly, the present invention preferably seeks to mitigate, alleviate or eliminate one or more of the above-identified deficiencies in the art and disadvantages singly or in any combination and solves at least the problems mentioned above, by providing a composition according to the appended patent claims.

According to one aspect of the invention, a composition is provided that allows for treatment of cancer in the urinary tract, prophylactic treatment of the urinary tract, and treatment of infection in the urinary tract. The composition comprises a nitric oxide (NO) eluting polymer arranged to contact the area to be treated, such that a therapeutic dose of nitric oxide is eluted from said nitric oxide eluting polymer to said area.

According to another aspect of the invention, a manufacturing process for such a composition is provided, wherein the process is a process for forming a composition that allows for target treatment of cancer in the urinary tract, prophylactic treatment of the urinary tract, and treatment of infection in the urinary tract. The process comprises selecting a nitric oxide eluting polymer, such as nano fibres, fibres, nano particles, or microspeheres, and deploying said nitric oxide eluting particles into forms such as nano-particles, micro-spheres, or powder to be comprised in said composition.

According to still another aspect of the present invention use of nitric oxide as a medicament to treat and/or prevent cancer in the urinary tract is provided.

The present invention has at least the advantage over the prior art that it presents the possibility to treat and/or prevent cancer in the urinary tract, prophylactic treatment of the urinary tract, and infection of the urinary tract, does not involve a surgical step, does not involve the use of chemotherapeutic materials, does not cause resistance against the active pharmaceutical substance, is easy to apply, provides improved circulation in form of a vasodilating effect, provides a painless treatment, and has fast inset of treatment effect, by the exposure of a urinary tract to NO, whereby a very effective anti-cancer therapy, prophylactic treatment, and/or anti-infection therapy is achievable.

### Description of Embodiments

The following description focuses on embodiments of the present invention applicable to a composition, in form of nano-particles, or micro-spheres, which allows for target treatment of cancer in the urinary tract, prophylactic treatment of the urinary tract, and treatment of infection in the urinary tract.

The term "urinary tract" is intended to be interpreted as including the urinary bladder, the ureter, the urethra, and the renal pelvis.

With regard to nitric oxide (nitrogen monoxide, NO), its physiological and pharmacological roles have attracted much attention and thus have been studied. NO is synthesized from L-arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is several orders higher, and that iNOS produces an extremely large amount of NO.

In the case of the generation of a large amount of NO as in the case of the production by iNOS, it is known that NO may react with active oxygen to attack exogenous microorganisms, such as bacterias and parasites, and cancer cells, but also to cause inflammation and tissue injury- On the other hand, in the case of the generation of a small amount of NO as in the case of the production by cNOS, it is considered that NO takes charge of various protective actions for a living body through the guanylate cyclase/GMP pathway (cGMP), such as vasodilator action, improvement of the blood circulation, antiplatelet-aggregating action, acceleration of the absorption in the digestive tract, regulation of renal function, neurotransmitter action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine), B-PEI (Branched PolyEthyleneImine), and PEI-C (PolyEthyleneImine Cellulose, which is a complex of polyethyleneimine and cellulose), which polymers have the advantage of being biocompatible, after the release of nitrogen oxide.

In one embodiment the polymers according to the present invention may be manufactured by electro spinning. Electro spinning is a process by which a polymer solution or melt is charged. At a characteristic voltage a fine jet of polymer releases from the surface in response to the tensile forces generated by interaction by an applied electric field with the electrical charge carried by the jet, on the way to the collector the jet stretches and solidifies, either by solvent evaporation or solidification. This process produces a non-woven mat of polymer fibres, such as nano-fibres. This jet of polymer fibres may be directed to a surface to be treated.

In one embodiment the polymers according to the present invention may be made into a solid powder, particles or granulate, before electrospinning through a reaction with a strong base to a salt- The salt can be modified with NO. Said salt could be integrated in a polymer carrier, such as any suitable polymer, which polymers are mentioned below in respect of polymers suitable for mixing with nitric oxide eluting polymer, that could be electro spun according to the procedure described above. The advantage with this method is that the loading with NO of the said polymer could be done before electrospinning.

Furthermore, US 6,382,526, US 6,520,425, and US 6,695,992 disclose processes and apparatuses for the production of such polymeric fibres. These techniques are generally based on gas stream spinning, also known within the fiber forming industry as air spinning, of liquids and/or solutions capable of forming fibers.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be spun onto the surface of medical devices such as implanted grafts, showing significant improvement of the healing process and reduced inflammation when implanting such devices- According to US-6,'737,447, a coating for permanently implanted medical devices provides nitric oxide delivery using nanofibers of linear poly(ethylenimine)-diazeniumdiolate. Linear poly(ethylenimine)diazeniumdiolate releases nitric oxide, (NO) in a controlled manner. Another advantage of L-PEI is that NO is released without any secondary products that could lead to undesired side effects.

In an embodiment of the invention the composition is embodied in form of nano-particles, or micro spheres. These nano-particles, or micro-spheres, may be formed from the NO-eluting polymers according to the present invention by spinning the NO eluting polymers into fibres, which fibres then are ground or milled into nano-particles or micro-spheres. These nano-particles, or micro-spheres, may the be injected into the urinary bladder, or applied in the urinary tract, in an amount sufficient to perform cancer treatment, prophylactic treatment, and treatment of infection. This injection may for example be done with a catheter through the urethra. When the nano-particles, or micro-spheres, get in contact with the moisture in the urinary bladder, the NO-eluting polymer starts to elute NO in the urinary bladder, to thereby subject the cancer cells and/or the infection to NO in such an amount as to achieve killing of said cancer cells and/or treating said infection.

In another embodiment the nano-paticles, micro-spheres, or powder of NO eluting polymer is mixed before injection with a solvent, said solvent having a proton donor capability. The solvent with a proton donor capability may for example be selected from the group; water and/or alcohol, such as ethanol. In this embodiment the elution of NO starts when the nano-particles, micro-spheres, or powder of NO eluting polymer mixed with the solvent with proton donor capability. Therefore, it is preferred that the mixing of nano-paticles, micro-spheres, or powder of NO eluting polymer and solvent is performed just prior to injection, to obtain as much elution of NO as possible inside the urinary bladder, or at other possible sites of therapy in the urinary tract. This injection is preferably performed with a catheter through the urethra.

In still another embodiment of the present invention the nano-paticles, micro-spheres, or powder of NO eluting polymer is mixed before injection with a hydrophobic solvent, for example hydrophobic cosmetic alcohol, such as lauryl alcohol. In this embodiment the elution of NO starts when the nano-paticles, micro-spheres, or powder of NO eluting polymer gets in contact with the water or moisture in the urinary tract. This embodiment provides the advantage of being able to store the NO eluting composition in a slurry without initiating the elution of NO. It may also be possible to regulate and/or control the elution of NO to the area to be treated, for example the urinary tract of the patient suffering from cancer in the urinary tract. This embodiment may also be combined with the possibility to mix the hydrophobic solvent/nitric oxide eluting polymer mixture with a pluronic, water based, gel. Upon mixing this mixture will form an emulsion and start to elute nitric oxide. This emulsion also presents the possibility to regulate and/or control the elution of nitric oxide.

In another embodiment the nano-particles, micro-spheres, or powder, according to the present invention may be formed from the NO-eluting polymers according to the present invention, encapsulated, or integrated, in any suitable material, such as polyvinylacetates, polylacticacids, starch, cellulose, polyhydroxyalkanoates, polycaprolactone, polyvinylalcohol, protein based plastics, gelatine, biogradable or biocompatible polymers, and other soluble plastics. The integration of, or encapsulation in, these materials is performed to regulate and/or control the elution of NO in the urinary tract, and to provide continuous exposure of the urinary tract to NO. The encapsulation may be such that the material breaks through reaction with the urine to thereby release the NO eluting polymer, which in contact with the water or moisture in the urinary tract starts to elute NO in the urinary tract of the patient suffering from cancer and/or infection, and/or prophylactic treatment in respect of said disorders, in said urinary tract.

In another embodiment of the present invention the nano-particles, or micro-spheres, or ground/pulverized NO-eluting polymer, may be encapsulated in a suitable material, such as gelatine, starch, cellulose etc, to be introduced into the urinary tract, such as the urinary bladder, as a capsule. When the thus obtained capsule reaches the urinary bladder, the gelatine, starch, cellulose, etc., dissolves and the nano-particles, or micro-spheres, starts to elute NO to the urinary bladder.

In another embodiment of the present invention the nano-particles, or micro-spheres, or ground/pulverized NO-eluting polymer are/is compressed into a pill, tablet or pellet, which pill, tablet or pellet, then is introduced into the urinary tract, such as the urinary bladder, of the patient suffering from cancer in the urinary tract. When the pill, tablet, or pellet, has been introduced, an effective anti-cancer effect is initiated in said urinary tract, such as the urinary bladder, when the water or moisture in the urinary tract initiates elution of nitric oxide.

In yet another embodiment of the present invention the NO-eluting composition may be combined with, or acting as a booster for, pharmaceuticals, chemotherapeutic agent, vitamins, nicotin, nitroglycerin etc. This embodiment presents a composition with the advantage of combining two therapeutic treatments, of significant value, in one treatment. A specific example of this embodiment is a combination of the composition according to the present invention and another active substances in respect of cancer, such as a chemotherapeutic substance. Hence, a synergetic effect may be achieved by such substances when NO that is eluted from the composition. NO has for instance a vasodilatory effect on the region where the composition acts. Thereby, the NO eluting composition in this embodiment may also facilitate the chemotherapeutic action of said chemotherapeutic substances, and hence achieving a synergistic effect. Vasodilated tissue is more susceptible to certain medications and thus more easily treated by the medical preparations and still NO has in addition to that the anti-cancer effect. Also, nitric oxide has an immunomodulating, cytotoxic and cytostatic effect. Nitric oxide acts as a signalling molecule in the human immune system. Thereby, nitric oxide can stimulate or inhibit different cells of the immune system depending on dose. This may in this embodiment of the present invention be exploited to boost another chemotherapeutic substance or to counteract adverse effects of a chemotherapeutic substance. Furthermore, it happens that cancerous cells become resistant to a chemotherapeutic substance. In this context this embodiment of the present invention combines the cytotoxic and/or cytostatic effect with the effect of said chemotherapeutic substance with a different mechanism of action to thereby achieve that the cancer cells will be less likely to develop resistance to said chemotherapeutic substance. Hence, an unexpected surprisingly effective treatment is provided.

Also, the effect of antibiotics in respect of treating infections, such as infections caused by bacteria and/or fungi, may be boosted and/or amplified by the use of nitric oxide in the same way as chemotherapeutic substances.

Urine is a unique environment in respect of treatment with nitric oxide, since the end oxidation product of nitric oxide is NO₂⁻. In almost all other biological tissues nitric oxide is oxidized to NO₃⁻, since there is haemoproteins, such as haemoglobin, present. It has been shown that NO₂⁻ may be reduced to NO in urine if pH is lowered and an antioxidant, such as ascorbic acid, is added.

Therefore, in one embodiment of the present invention, an antioxidant, such as ascorbic acid, is included in the NO eluting composition. A ten fold increase of this effect is obtained if an antioxidant, such as ascorbic acid, is present.

The effect mentioned above, in respect of the unique environment of urine in treatment with NO, results in that very high concentrations of nitric oxide may be obtained during long periods of time in the urinary bladder, since the nitric oxide that is eluted in urinary bladder is first oxidized to NO₂⁻, which in return is reduced back to nitric oxide. This effect may be observed already at a pH of 7.5, but is strongly amplified at lower pH. A substantial amount of formed nitric oxide is obtained from NO₂⁻ in urine at a pH of 4 to 5. All in all this results in the possibility to substantially potentiate the effects of a nitric oxide eluting system by simultaneously lowering the pH and adding an antioxidant.

Therefore, in another embodiment of the present invention, pH is lowered in the urinary tract during treatment with the NO eluting composition. The lowering of the pH may for example be accomplished by addition of ammonium chloride and/or ammonium sulphate.

In another embodiment of the composition according to the present invention, the nano-particles, micro-spheres, and/or powder, are integrated in a gel. It may also be integrated in a hydrogel, which is mixed directly before use. This gel is then introduced into the urinary bladder by injection through the urethra, and the composition elutes NO when the gel, or hydrogel, reaches the urinary bladder. This gel may also be applied in for example the urethra or other sites of the urinary tract.

In yet another embodiment of the present invention the NO eluting polymer is included in a catheter and/or catheter balloon to thereby provide the possibility to elute nitric oxide to the area of application of said catheter or catheter balloon. If such a catheter balloon is manufactured of silicone nitric oxide will be able to elute from the inside of the catheter balloon to the vicinity of the catheter balloon through the silicone wall of the catheter balloon.

In yet another embodiment of the present invention the composition is in form of a foam or cream.

When the NO-eluting composition according to the present invention gets in contact with the moisture or water in the urinary tract, the NO-eluting composition starts to release NO to the urinary tract to be treated. This composition does not cause resistance against nitric oxide (NO), is easy to apply, provides a painless treatment, has fast inset of treating and/or preventing anti-cancer effect and/or anti-infection effect.

In embodiments of the present invention the NO eluting composition may be maintained in the urinary tract, such as the urinary bladder, of the patient suffering from cancer in said urinary tract until a sufficient time of treatment has been obtained. Then the composition may be discharged by urinating action of the patient or by discharging the composition through suction from the urinary bladder.

In one embodiment of the present invention ethambutol is used as the carrier of nitric oxide, to thereby constitute a nitric oxide eluting substance. In this embodiment nitric oxide is eluted in the same way as with the nitric oxide eluting polymers discussed above.

The treatment with the composition according to the present invention is very effective in respect of treating both cancer in the epithelial cells (the internal lining), such as superficial cancer in the urinary bladder, and cancer in the muscle layer of the urinary bladder. This combinatory effect is achieved through the anti-cancer effect of NO and the vasodilating effect of NO.

Other NO elution regulating and/or controlling polymers, according to the invention, may be polyesters, polyamides, polyethers, polyurethanes, polycarbonates, polyvinylacetates, polylacticacids, starch, cellulose, polyhydroxyalkanoates, polypropylene, cotton, polyesters, polycaprolactone, polyvinylalcohol, polyacrylonitrile, polystyrene, poly(acrylic acid), polypropylene, protein based plastics, gelatine, and other biocompatible polymers. The NO-eluting polymer, such as L-PEI, may be integrated in, spun together with, or spun on top of, any of these materials.

The device according to the present invention elutes nitric oxide (NO) from said eluting polymer in a therapeutic dose in the urinary tract, such as between 0.001 to 5000 ppm, such as 0.01 to 3000 ppm, such as 0.1 to 1000 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm. The concentration may vary widely depending on where the concentration is measured. If the concentration is measured close to the actual NO eluting polymer the concentration may be as high as thousands of ppm, while the concentration inside the tissue in this case often is considerably lower, such as between 1 to 1000 ppm.

The NO-eluting polymers in the composition according to the present invention may be combined with silver, such as hydroactivated silver. The integration of silver in the composition according to the present invention gives the healing process an extra boost. Preferably the silver is releasable from the composition in the form of silver ions.

In the embodiments of the present invention it may be suitable to control or regulate the time span of NO release from the composition according to the invention. This may be accomplished by integrating other polymers or materials in said composition. These polymers or materials may be chosen from any suitable material or polymer, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics

The composition according to the present invention may comprise polymers manufactured by, for example electro spinning of L-PEI or other polymers comprising L-PEI or being arranged in combination with L-PEI. L-PEI is the charged at a characteristic voltage, and a fine jet of L-PEI releases as a bundle of L-PEI polymer fibres. The L-PEI polymer fibres may be spun onto any suitable material in respect of the present invention. The electro spun fibres of L-PEI then attach on said material from which the nano-particles, micro-spheres, or powder are/is formed. Such fibres are also easily further processed to other forms, such as the above mentioned powder, which simply is obtainable by applying a shredder or blender type apparatus to the fibres until a powder of desired granulation size is received.

In one embodiment the polymers according to the present invention may be made into a solid powder, particles or granulate, before electrospinning through a reaction with a strong base to a salt. The salt can be modified with NO. Said salt could be integrated in a polymer carrier, such as any suitable polymer, which polymers are mentioned below in respect of polymers suitable for mixing with nitric oxide eluting polymer, that could be electro spun according to the procedure described above. The advantage with this method is that the loading with NO of the said polymer could be done before electrospinning.

It is of course possible to electro spin the other NO-eluting polymers, according to above, on the thus obtained NO eluting polymers, which may be comprised in the compositions according to the invention, while still be inside the scope of the present invention.

In one embodiment the NO-eluting polymers according to the present invention are electro spun in such way that pure NO-eluting polymer fibres may be obtained.

Gas stream spinning, or air spinning, of said NO-eluting polymers onto material which combination of NO-eluting polymer and other material may be comprised in the composition according to the present invention, is also within the scope of an embodiment of the manufacturing method according to the present invention.

The manufacturing process according to the present invention presents the advantages of providing compositions with large contact surface of the NO-eluting polymer fibres and with the area to be treated, effective use of NO-eluting polymer, and a cost effective way of producing the composition according to the present invention.

Hereinafter, some potential uses of the present invention are described:

A method of therapeutical treatment of cancer in the urinary tract by means of a composition comprising a nitric oxide (NO) eluting polymer configured for eluting a therapeutic dosage of nitrogen oxide (NO) when used for said treatment, comprising exposing said treatment site of said cancer in the urinary tract to said nitric oxide when said polymer in use elutes nitrogen oxide (NO) by eluting a therapeutic dose of nitric oxide from said nitric oxide eluting polymer to said urinary tract.

The method according to the above, wherein said method comprises applying nano-particles, micro-spheres, or powder as a pill, a tablet, a pellet, a capsule, composition, a gel, a hydrogel, a foam, a cream, and/or granules, to said site for said exposure.

Use of nitric oxide (NO) in a therapeutic dose for therapeutically treating cancer in the urinary tract.

The invention may be implemented in any suitable form. The elements and components of the embodiments according to the invention may be physically, functionally, and logically implemented in any suitable way. Indeed, the functionality may be implemented in a single unit, in a plurality of units, or as part of other functional units.

Although the present invention has been described above with reference to specific embodiments, it is not intended to be limited to the specific form set forth herein. Rather, the invention is limited only by the accompanying claims and, other embodiments than the specific above are equally possible within the scope of these appended claims.

In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A composition configured to therapeutically target treat, prophylactically treat and/or prevent cancer and/or infection in a urinary tract, wherein
said composition elutes nitric oxide (NO) in a therapeutic dosage, and said composition being configured for exposing the urinary tract to said eluted NO.

2. Composition according to claim 1, wherein said composition comprises a nitric oxide (NO) eluting polymer configured for eluting a therapeutic dosage of nitrogen oxide (NO)configured for exposing an area of treatment in the urinary tract to said nitric oxide when said polymer in use elutes nitrogen oxide (NO).

3. Composition according to claim 2, wherein said polymer is selected from the group comprising polyalkyleneimines, S-nitrosylated polymer, and poly(alkylenimine)diazeniumdiolates, or any combinations thereof.

4. Composition according to claim 2, wherein said polymer is L-PEI (linear polyethyleneimine), loaded with nitric oxide (NO), arranged for release of the nitric oxide (NO) at said urinary tract.

5. Composition according to claim 1, wherein said composition comprises an antioxidant.

6. Composition according to claim 5, wherein said antioxidant is ascorbic acid.

7. Composition according to claim 1, wherein said composition comprises a pH lowering substance.

8. Composition according to claim 7, wherein said pH lowering substance is ammonium chloride and/or ammonium sulphate.

9. Composition according to claim 1, wherein said composition comprises nitric oxide (NO) eluting ethambutol.

10. Composition according to claim 1, in a form selected from the group consisting of powder, nano-particles or micro-spheres, pill, tablet, pellet, gel, hydrogel, foam, cream, granules, and capsule, or combinations thereof.

11. Composition according to claim 1, wherein said composition elutes NO when subjected to moisture or water.

12. Composition according to claim 2, wherein said polymer comprises silver, configured for said therapeutical target treatment, prophylactical treatment and/or prevention of cancer and/or infection in the urinary tract.

13. Composition according to claim 2, wherein said polymer is acting as a booster for pharmaceuticals, vitamins, and drugs, or combinations thereof, combined with silver.

14. Composition according to claim 1, wherein said composition is combined with a chemotherapeutic agent.

15. Composition according to claim 1, wherein said composition comprises a hydrophobic alcohol.

16. Composition according to claim 15, wherein said hydrophobic alcohol is lauryl alcohol.

17. Composition according to claim 10, wherein said nano-particles, micro-spheres, or granules, are encapsulated, or integrated, in a material, selected from the group consisting of polyvinylacetates, polylacticacids, starch, cellulose, polyhydroxyalkanoates, polyesters, polycaprolactone, polyvinylalcohol, protein based plastics, and/or gelatine, polyesters, polyamides, polyethers, polyurethanes, polycarbonates, polyvinylacetates, polylacticacids, starch, cellulose, polyhydroxyalkanoates, polypropylene, cotton, polyesters, polycaprolactone, polyvinylalcohol, polyacrylonitrile, polystyrene, poly(acrylic acid), polypropylene, protein based plastics, gelatine, and other biocompatible polymers, and combinations thereof, for regulating and/or controlling elution of NO.

18. Composition according to claim 10, wherein said nano-particles, or micro-spheres, are integrated in a gel, hydrogel, pill, tablet, capsule or foam, and combinations thereof.

19. Composition according to claim 10, wherein said granules are of a material, selected from the group consisting of polyesters, polyamides, polyethers, polyurethanes, polycarbonates, polyvinylacetates, polylacticacids, starch, cellulose, polyhydroxyalkanoates, polypropylene, cotton, polyesters, polycaprolactone, polyvinylalcohol, polyacrylonitrile, polystyrene, poly(acrylic acid), polypropylene, protein based plastics, gelatine, and other biocompatible polymers, and combinations thereof, integrated, or covered, with said NO-eluting polymer.

20. A manufacturing process for a composition according to claim 2, comprising
selecting a plurality of nitric oxide eluting polymeric particles, preferably nano fibres, nano particles or micro spheres, and
deploying said nitric oxide eluting polymeric particles into a suitable form or as a coating onto a carrier, to form a material comprised in said composition,
said deploying comprises electro, air, or gas stream spinning of said particles.

21. Use of a nitric oxide (NO) eluting polymer for the manufacture of a composition for the treatment and/or prevention of cancer and/or infection in the urinary tract wherein
nitric oxide is loaded to said composition so that said composition elutes nitric oxide (NO) from said eluting polymer in a therapeutic dose when used.

22. Use according to claim 21, wherein said therapeutic dose is 0.001 to 5000 ppm, such as 0.01 to 3000 ppm, such as 0.1 to 1000 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

23. Nitric oxide (NO) for use as a medicament in the treatment of cancer in the urinary tract.

24. Nitric oxide (NO) for use as a medicament in the treatment of cancer in the epithelial cells in the urinary tract.

25. Nitric oxide (NO) for use as a medicament in the treatment of infection in the urinary tract.
